**Europäisches Patentamt**

**European Patent Office**

**Office européen des brevets**

(19)

(11) Veröffentlichungsnummer : **0 397 113 B1**

(12)

# EUROPÄISCHE PATENTSCHRIFT

(45) Veröffentlichungstag der Patentschrift :
**18.11.93 Patentblatt 93/46**

(51) Int. Cl.⁵ : **G01N 33/544,** G01N 33/546,
G01N 33/548, G01N 33/573

(21) Anmeldenummer : **90108656.1**

(22) Anmeldetag : **08.05.90**

(54) **Verfahren zum Nachweis spezifisch bindefähiger Substanzen in Körperflüssigkeiten.**

(30) Priorität : **09.05.89 DE 3915135**

(43) Veröffentlichungstag der Anmeldung :
**14.11.90 Patentblatt 90/46**

(45) Bekanntmachung des Hinweises auf die
Patenterteilung :
**18.11.93 Patentblatt 93/46**

(84) Benannte Vertragsstaaten :
**AT BE CH DE DK ES FR GB GR IT LI LU NL SE**

(56) Entgegenhaltungen :
**EP-A- 0 138 297**
**EP-A- 0 269 092**
**EP-A- 0 353 895**
**EP-A- 0 356 964**
**FR-A- 2 624 613**
**US-A- 4 282 287**
**US-A- 4 550 075**

(73) Patentinhaber : **BOEHRINGER MANNHEIM
GMBH
D-68298 Mannheim (DE)**

(72) Erfinder : **Ofenloch-Hähnle, Beatus, Dr.
Offenbachstrasse 41
D-8000 München 60 (DE)**
Erfinder : **Berger, Michael, Dr.
Knappenstrasse 16
D-8122 Penzberg (DE)**
Erfinder : **Deger, Arno, Dr.
Föhrenstrasse 3
D-8124 Seeshaupt (DE)**

(74) Vertreter : **Huber, Bernhard, Dipl.-Chem. et al
Patentanwälte H. Weickmann, Dr. K. Fincke
F.A. Weickmann, B. Huber Dr. H. Liska, Dr. J.
Prechtel, Dr. B. Böhm Postfach 86 08 20
D-81635 München (DE)**

## Beschreibung

Die Erfindung betrifft ein Verfahren zum Nachweis spezifisch bindefähiger Substanzen in Körperflüssigkeiten, nach dem Prinzip des Immunoassay unter Verwendung von Liganden-Konjugaten zur Markierung, Immobilisierung oder Komplexierung die die für die Liganden-Konjugate verwendeten Liganden in freier Form enthalten.

In der klinischen Diagnostik werden eine Vielzahl von Tests zum Nachweis von spezifisch bindefähigen Substanzen in Körperflüssigkeiten durchgeführt. Viele Bestimmungen basieren auf dem Prinzip des homogenen oder heterogenen Immunoassays, wobei in der Regel mindestens zwei Rezeptoren verwendet werden, von denen einer markiert ist und der andere immobilisiert oder immobilisierbar ist. In neuerer Zeit werden für die markierten bzw. immobilisierten oder immobilisierbaren Rezeptoren häufig Konjugate verwendet, bei denen die Bindung des mit der zu bestimmenden Substanz spezifisch bindefähigen Immunpartners an die Markierung oder an die feste Phase über die Wechselwirkung zwischen einem Liganden und einem für diesen Liganden spezifischen Bindungspartner erfolgt. Der Ligand ist in der Regel ein kleineres Molekül, z.B. ein Hapten. Da die Bindungskraft zwischen Biotin und Streptavidin bzw. Avidin eine der höchsten Konstanten für biologische Moleküle aufweist, wird Biotin sehr häufig als Ligand verwendet und Streptavidin als dafür spezifischer Bindungspartner. Neben den vielen Vorteilen, die die Verwendung dieses Paares bringt, hat sie den Nachteil, daß Biotin ein in Körperflüssigkeiten ubiquitär vorkommendes Molekül ist. Daher ist in den Probelösungen Biotin in freier Form vorhanden, besetzt Bindungsstellen und kann daher zu Verfälschungen des Tests führen. Besonders kritisch ist dies bei Patienten, die hochdosierte Biotinmengen einnehmen. Man geht davon aus, daß Biotin in einer Menge von mehr als 30 ng/ml Probe bereits zu verfälschten Ergebnissen führt. Bei mit Biotin behandelten Patienten können Serumwerte von bis zu 180 ng/ml und Dauerwerte von etwa 70 ng/ml auftreten. Man sucht daher nach einer Methode, um das Biotin so zu beseitigen, daß eine Störung des Tests dadurch nicht erfolgt. Dieselbe Problematik tritt auf, wenn man andere Haptene wie beispielsweise Digoxigenin als Ligand verwendet, die gleichzeitig in der Therapie angewendet werden und daher ebenfalls in Körperflüssigkeiten vorhanden sein können und dort eine Störung bewirken.

Es war daher Aufgabe der Erfindung, ein Verfahren zum Nachweis von Substanzen in Körperflüssigkeiten, die störende Liganden enthalten, zur Verfügung zu stellen, um diese Liganden so abzufangen, daß das Nachweisverfahren dadurch nicht gestört wird.

Diese Aufgabe wird gelöst durch ein Verfahren zum Nachweis spezifisch bindefähiger Substanzen in Körperflüssigkeiten nach dem Prinzip des Immunoassay unter Verwendung von Ligandenkonjugaten zur Markierung, Immobilisierung oder Komplexierung, die die für die Ligandenkonjugate verwendeten Liganden in freier Form enthalten, das dadurch gekennzeichnet ist, daß man die in freier Form vorliegenden Liganden der immunologischen Reaktion entzieht, indem man die Probelösung mit Polymerteilchen inkubiert, die als Kern ein Polymer, das eine Vielzahl von Bindungsstellen für den Liganden aufweist und als Umhüllung mindestens eine Schicht aus Protein, Peptid, Nukleinsäurepolymer, Kohlenhydrat und/oder einem Copolymerisat aus Kohlenhydrat und Aminosäuren haben, wobei die Schichtdicke der Umhüllung so eingestellt wird, daß die Beschichtung durchlässig für den freien Liganden, nicht jedoch für das Ligandenkonjugat ist.

Überraschenderweise gelingt es mit den erfindungsgemäß verwendeten Polymerteilchen, die störenden Liganden aus der Lösung abzufangen. Die Polymerteilchen sind so aufgebaut, daß nur der freie Ligand gebunden werden kann, nicht jedoch ein Ligandkonjugat, dessen Molekulargewicht um mehr als den Faktor 2 höher ist als das des Liganden. Auf diese Weise gelingt es auch bei hoch mit freiem Liganden belasteten Probelösungen, die Störung durch den Liganden zu beseitigen, ohne die Funktion des Ligandenkonjugats im Testsystem negativ zu beeinflussen.

Die erfindungsgemäß durchgeführte Entfernung der freien Liganden gelingt für alle Varianten von Nachweisverfahren nach dem Prinzip des Immunoassay, bei denen ein Ligandkonjugat zur Markierung, Immobilisierung oder Komplexierung verwendet wird. Als Ligand wird hierbei ein Partner eines spezifisch bindenden Paares bezeichnet, der ein relativ niedriges Molekulargewicht aufweist und in Form von Konjugaten mit Markierungen wie z.B. Enzymen oder fluoreszierenden Substanzen oder gebunden an einen Antikörper oder dessen Fragment, zur Bindung des Antikörpers an die feste Phase verwendet wird. In der Regel werden als Liganden Haptene, niedrigmolekulare Antigene sowie Antikörper und deren Fragmente angesehen. Beispiele sind Biotin und Digoxigenin. Der Ligand zeigt weder mit der nachzuweisenden Substanz noch mit dem Rezeptor Kreuzreaktion.

Beispiele für Nachweisverfahren, bei denen das erfindungsgemäße Prinzip angewendet werden kann, sind Sandwich-Tests und kompetitive Tests im 1-Schritt- oder 2-Schritt-Verfahren. Ein Beispiel für eine geeignete Ausführungsform ist ein Sandwich-Test im 1-Schritt-Verfahren, bei dem an einer Festphase Partner eines spezifischen Bindungssystems immobilisiert sind und die nachzuweisende Substanz mit einem markierten Rezeptor sowie einem unmarkierten Rezeptor-Konjugat, das den anderen Bindungspartner enthält, inkubiert wird. Der sich bildende Komplex aus zu bestimmender Substanz, markiertem Rezeptor und Rezeptor mit Bindungspartner bindet dann aufgrund der spezifischen Bindefähigkeit an den anderen Partner, der an der Festphase immobilisiert ist, so daß auf diese Weise der gesamte Komplex immobilisiert wird. Nach Trennung der Phasen kann dann in einer der beiden Phasen die Markierung gemessen werden.

Um den in freier Form vorliegenden und damit die Bestimmung störenden Liganden der immunologischen Reaktion zu entziehen, wird die Probelösung mit Polymerteilchen inkubiert, die spezifisch die freie Form des Liganden binden und aus einem Kern und einer Umhüllung bestehen.

Der Kern liefert die Bindungsstellen für den Liganden und die Umhüllung bildet die Schranke zur Selektion der Liganden von den Ligandenkonjugaten.

In einer bevorzugten Ausführungsform sind die Polymerteilchen wasserlöslich und befinden sich in der Inkubationslösung, die Probe und Reagenzien enthält.

Der Kern weist eine Vielzahl von Bindungsstellen für den Liganden auf. Er kann entweder ein homogenes oder heterogenes Polymer von mit dem Liganden bindefähigen Substanzen sein oder aber wiederum aus einem inerten Kern und einer Hülle aus bindefähigen Substanzen bestehen. Als inert werden hierbei Substanzen bezeichnet, die im Hinblick auf die immunologische Reaktion und die Nachweisreaktion inert sind.

Die Bindungsstellen im Kern für den Liganden werden durch eine mit dem Liganden spezifisch bindefähige Substanz geliefert. Diese spezifisch bindefähige Substanz kann dabei der andere Partner eines spezifisch bindenden Paares sein und kann identisch sein mit dem zur Bindung des Liganden verwendeten Partner, der in immobilisierter oder markierter Form vorliegt. Geeignete Bindungspaare sind z.B. Biotin-Avidin, Biotin-Streptavidin, Antigen-Antikörper, Hapten-Antikörper, Protein A-Immun-$\gamma$-Globulin, Protein G-Immun-$\gamma$-Globulin sowie Hapten-Bindeprotein, sowie die jeweils bindefähigen Derivate. Das Polymer aus den mit dem Liganden bindefähigen Substanzen kann hergestellt werden durch Vernetzung der einzelnen Bindepartner miteinander oder mit einem inerten Molekül. Die einzelnen Bindepartner können über homo- oder heterobi- oder polyvalente Linker miteinander verbunden werden. Bevorzugt wird die Vernetzung mit bivalenten Linkern durchgeführt, da dies eine leichtere Steuerung des Polymerisationsgrades ermöglicht. Ebenso geeignet sind jedoch auch polyvalente Linker. Als Linker können solche Verbindungen verwendet werden, die reaktionsfähige Gruppen aufweisen, die in wäßriger Lösung mit den funktionellen Gruppen der spezifisch bindefähigen Partner unter Ausbildung einer kovalenten Bindung zu reagieren vermögen. Dem Fachmann ist eine große Anzahl hierfür geeigneter bifunktioneller oder polyfunktioneller Linker bekannt. Typische Beispiele für im Rahmen der Erfindung gut geeignete homo- oder heterobifunktionelle und trifunktionelle Linker sind in der nachstehenden Tabelle 1 aufgeführt.

T a b e l l e   1

| Kurzbe-zeichnung | Chemische Bezeichnung |
|---|---|
| SPDP | N-succinimidyl 3-(2-pyridyldithio)-propionat |
| EADB | Ethyl 4-azidophenyl-1,4-dithiobutyrimidat · HCl |
| FNPA | 4-Fluoro-3-nitrophenylazid |
| HSAB | N-Hydroxysuccinimidyl-4-azidobenzoat |
| MABI | Methyl-4-azidobenzoimidat · HCl |
| MBS | m-Maleimidobenzoyl-N-hydroxysuccinimid-ester |
| NHS-ASA | N-Hydroxysuccinimidyl-4-azidosalicylsäure |
| MHS | Maleimidohexanoyl-N-Hydroxysuccinimidester |
| PNP-DTP | p-Nitrophenyl-2-diazo-3,3,3-trifluoropropionat |
| SADP | N-Succinimidyl(4-azidophenyl)1,3'-dithiopropionat |
| SAND | Sulfosuccinimidyl-2-(m-azido-o-nitrobenzamido)-ethyl-1,3'-dithiopropionat |
| SANPAH | N-succinimidyl-6(4'-azido-2'-nitrophenyl-amino)hexanoat |
| SASD | Sulfosuccinimidyl 2-(p-azidosalicylamido)ethyl-1,3'-dithiopropionat |
| SIAB | N-Succinimidyl(4-iodoacetyl)aminobenzoat |
| SMCC | Succinimidyl-4-(N-maleinimidoethyl)cyclohexan-1-carboxylat |
| SMPB | Succinimidyl-4-(p-maleimidophenyl)butyrat |
| DSS | Disuccinimidylsuberat |
| DMS | Dimethylsuberimidat |
| Traut's Reagenz | 2-Iminothiolan |
| | 2,4,6 Trichlor-s-triazin |
| SAMBA | S'-Acetyl-mercaptobernsteinsäureanhydrid |
| SATA | N-succinimidyl-S-acetylthioacetat |

Zur Durchführung der Vernetzung kann eine Lösung der Bindepartner mit den Linkermolekülen versetzt werden unter Bedingungen, die direkt zur Vernetzung führen. Das Ausmaß der Vernetzung wird in diesem Falle durch die Menge an zugegebenem Linker gesteuert. In einer weiteren bevorzugten Ausführungsform wird der Bindepartner mit geeigneten bindefähigen Komponenten, die in bezug auf den Liganden und auf die zu bestimmende Verbindung inert sind, vernetzt. Hierzu geeignet ist beispielsweise ein lösliches Protein, insbesondere Rinderserumalbumin oder Humanserumalbumin.

Der Kern kann ganz aus dem Polymer der Bindepartner bestehen. Er kann ebenso einen inerten Kern aufweisen, der beispielsweise aus Polystyrol oder Dextran bestehen kann und mit dem Polymer aus den mit dem Liganden bindefähigen Substanzen umhüllt ist.

Bei dem am häufigsten verwendeten Liganden Biotin wird für das Kernpolymer ein Polymerisat aus Antibiotin-Antikörpern oder besonders bevorzugt aus Avidin oder Streptavidin verwendet. Bei der Verwendung von Digoxigenin als Ligand enthält das Kernpolymer bevorzugt Anti-Digoxigenin-Antikörper oder deren Fragmente. Ist Streptavidin oder Avidin der Ligand, so enthält das Kernpolymer Biotinmoleküle oder deren mit Streptavidin bzw. Avidin bindefähigen Derivate wie z.B. Iminobiotin oder Biocytin.

Der Kern wird von einer Umhüllung umgeben, die so ausgebildet ist, daß die Beschichtung durchlässig für den freien Liganden, nicht jedoch für das Liganden-Konjugat ist. Die Umhüllung besteht aus mindestens einer Schicht aus Protein, Peptid, Nukleinsäurepolymer, Kohlenhydrat und/oder einem Copolymerisat aus Kohlenhydrat und Aminosäuren. Diese Komponenten können auch in derivatisierter Form verwendet werden.

Die Größe des Kerns ist an sich unkritisch und im wesentlichen durch die Löslichkeit der Teilchen im Molekulargewicht begrenzt. Für eine Bindung der in freier Form vorkommenden Liganden ist eine ausreichende Anzahl Bindungsstellen im Kern notwendig. Es hat sich gezeigt, daß einige wenige Bindungsstellen im Kern bereits eine merkliche Entstörung bewirken. Gute Entstörung findet man ab ca. 10 Bindungsstellen, besonders gute Entstörung ab ca. 50 Bindungsstellen und mehr.

Bevorzugt werden zur Bildung der Umhüllung polymeres Rinderserumalbumin, polymere Fab-Fragmente oder Aminodextranpolymer verwendet. Die Herstellung der Polymere erfolgt nach bekannten Methoden durch Vernetzung der einzelnen Komponenten. Die Schichtdicke wird dabei so eingestellt, daß die Beschichtung durchlässig für den freien Liganden, nicht jedoch für das Liganden-Konjugat ist.

Das Kernpolymer wird mit mindestens einer Schicht umhüllt. Bevorzugt wird die Umhüllung aus mindestens zwei Schichten, die gleich oder verschieden sein können, aufgebaut. Bevorzugt weist die Umhüllung eine schwamm- oder baumartige Struktur auf, die besonders gut eine selektive Durchlässigkeit ermöglicht. Sie entsteht z.B. bei der mehrfachen Umhüllung mit Rinderserumalbumin und/oder Fab'-Fragmenten von Antikörpern.

Die Beschichtung kann kovalent, adsorptiv oder durch Bindung über ein spezifisches Bindungspaar (z.B. Biotin/Avidin) als funktionale Verknüpfung erfolgen.

Mit dem erfindungsgemäßen Verfahren gelingt es, störende Liganden aus der Probelösung abzufangen. Die Entstörung gelingt bis in hohe Konzentrationsbereiche. So ist es möglich, aus Probelösungen, die bis zu 200 ng/ml Biotin enthalten, das Biotin nahezu vollständig zu beseitigen. Bei noch höher belasteten Proben gelingt eine zumindest weitgehende Beseitigung. Damit ermöglicht das Verfahren eine Verbesserung der Genauigkeit und Reproduzierbarkeit von Bestimmungen nach dem Prinzip des Immunoassays.

Gegenstand der Erfindung ist auch ein Ligandfänger, der dadurch gekennzeichnet ist, daß er aus einem wasserlöslichen polymeren Rezeptor-Molekül, das Kern und Umhüllung umfaßt, besteht, wobei der Kern ein Polymer ist, das eine Vielzahl von Bindungsstellen für den Liganden aufweist und die Umhüllung aus mindestens einer Schicht aus Protein, Peptid, Nukleinsäurepolymer, Kohlenhydrat und/oder einem Copolymerisat aus Kohlenhydrat und Aminosäure besteht, wobei die Schichtdicke der Umhüllung so ist, daß die Beschichtung durchlässig für den freien Liganden, nicht jedoch für ein Ligandenkonjugat ist.

Die Erfindung wird durch die folgenden Figuren und Beispiele erläutert:

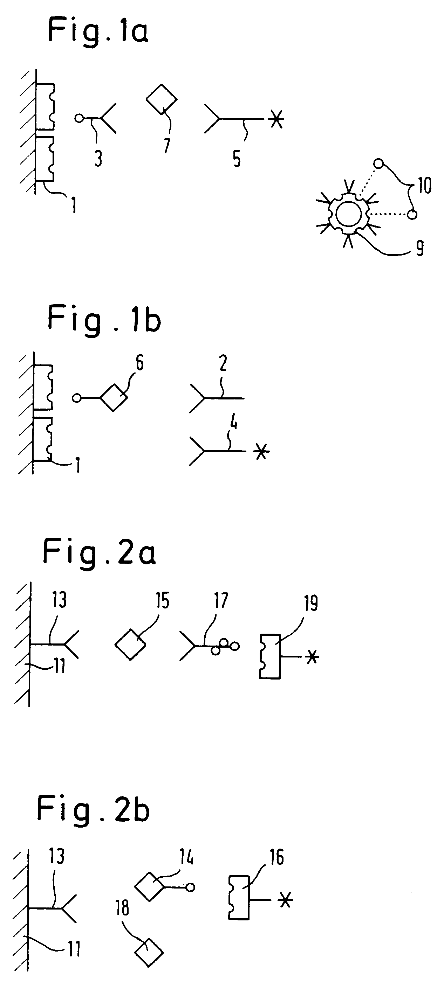

Fig. 1a      zeigt eine bevorzugte Ausführungsform des erfindungsgemäßen Verfahrens. Hier werden eine Festphase 1, an der Streptavidin immobilisiert ist, mit einem Rezeptor 3, der mit der zu bestimmenden Substanz 7 bindefähig ist und mit Biotin konjugiert ist, sowie einem markierten Rezeptor 5, der mit der zu bestimmenden Substanz 7 bindefähig ist, zusammen mit der Probelösung inkubiert. Die Lösung enthält weiterhin den erfindungsgemäßen Ligandenfänger 9. Bei der Reaktion bindet der Analyt 7, an dem der markierte Rezeptor 5 gebunden ist, über den Rezeptor 3 an die Festphase 1. In der Lösung vorhandenes Biotin 10 reagiert mit dem Ligandenfänger 9 und geht nicht in die immunologische Reaktion ein.

Fig. 1b      zeigt eine Verfahrensvariante eines kompetitiven Immunoassays. An eine Festphase 1 ist Streptavidin gebunden. Die Festphase wird inkubiert mit dem zu bestimmenden Antikörper 2 sowie einem dem zu bestimmenden Antikörper analogen Antikörper 4, der eine Markierung trägt und weiterhin einem mit Analyt und markiertem Rezeptor bindefähigen Antigen 6, das mit Biotin konjugiert ist. Bei der Reaktion konkurrieren zu bestimmender Antikörper 2 und markierter Rezeptor 4 um die Bindung an das Antigen 6. Die gebildeten Komplexe aus Antikörper und Antigen binden über Biotin an die mit Streptavidin beschichtete Festphase.

Die Fig. 2a und 2b zeigen weitere Varianten des erfindungsgemäßen Verfahrens.

Fig. 2a      zeigt eine Ausführungsform, bei der an einer Festphase 11 ein Antikörper 13, der mit der zu bestimmenden Substanz 15 bindefähig ist, immobilisiert ist. Mit der Probelösung, die die zu bestimmende Substanz 15 enthält, wird ein Rezeptor 17 inkubiert, der mit der zu bestimmenden Substanz 15 bindefähig ist und mit Biotin konjugiert ist. Weiterhin in der Probelösung vorhanden ist ein Konjugat 19 aus einer Markierung und Streptavidin. Bei der immunologischen Reaktion reagiert Rezeptor 17 mit der zu bestimmenden Substanz 15 und wird über den Antikörper 13 an der Festphase immobilisiert. Die Markierung wird gebunden über das in Rezeptor 19 enthaltende Streptavidin und das in Rezeptor 17 enthaltende Biotin.

Fig. 2b zeigt als weitere Variante ein kompetitives Verfahren. An der Festphase 11 ist ein mit der zu bestimmenden Substanz bindefähiger Antikörper 13 immobilisiert. Der Probelösung wird ein Konjugat 14 zugegeben, das aus der zu bestimmenden Substanz und Biotin besteht sowie ein Konjugat aus einer Markierung und Streptavidin. Bei der immunologischen Reaktion konkurrieren zu bestimmende Substanz 18 und biotinhaltiger Rezeptor um die Bindung an den festphasengebundenen Antikörper. Die Bindung der Markierung erfolgt über das in dem markierten Rezeptor enthaltene Streptavidin an das biotinhaltige Konjugat.

**Beispiel 1**

Herstellung von Polystreptavidin (SA(P))

Herstellung von Maleiniminohexanoylstreptavidin (SA-MH): 500 mg Streptavidin werden in 50 ml 50 mmol/l Kaliumphosphatpuffer (KPP) und 100 mmol/l NaCl, pH 6,6, gelöst und mit 25 mg Maleinimidohexanoyl-N-hydroxysuccinimidester (MHS), vorgelöst in DMSO, 2 Stunden bei 4°C inkubiert. Danach wird gegen 10 mmol/l KPP/50 mmol/l NaCl, pH 6,2, dialysiert.

Herstellung von mit SAMBA derivatisiertem Streptavidin (SA-SAMB): Weitere 500 mg Streptavidin werden in 50 mmol/l KPP/100 mmol/l NaCl, pH 7,8, gelöst, mit 29 mg SAMBA, vorgelöst in DMSO, versetzt und 3,5 Stunden unter Rühren bei 25°C inkubiert. Danach wird die Reaktionsmischung gegen 50 mmol/l KPP/100 mmol/l NaCl/1 mmol/l EDTA, pH 6,0, dialysiert.

Kopplung von SA-MH und SA-SAMB: 50 ml der dialysierten SA-SAMB-Lösung werden mit 275 $\mu$l 1 mol/l Hydroxylamin-Lösung versetzt und 30 Minuten bei 25°C unter Rühren inkubiert. Danach wird die Reaktionsmischung zweimal eine Stunde gegen je 2 l 50 mmol/l KPP/100 mmol/l NaCl/2 mmol/l EDTA, pH 6,5, dialysiert. Das Dialysat wird mit 110 ml des Dialysepuffers versetzt und 55 ml der dialysierten SA-MH-Lösung zugegeben. Die Mischung wird 2 bis 4 Stunden bei 25°C unter Rühren inkubiert und der Vernetzungsgrad in halbstündigen Abständen mittels FPLC-Gelfiltration überprüft. Ist ein mittleres apparentes Molekulargewicht von 700 bis 800 kD erreicht, wird die Vernetzung durch Zugabe von Cystein (ad 1 mmol/l, 30 Minuten, 25°C) und Iodacetamid (ad 5 mmol/l, 30 Minuten, 25°C) gestoppt. Das erhaltene Streptavidinpolymerisat SA(P) wird durch Gelfiltration an Superose 6 in 50 mmol/l KPP/100 mmol/l NaCl/l % Saccharose, pH 6,8, aufgereinigt.

Die SA(P) enthaltenden Fraktionen werden gesammelt, mittels Ultrafiltration auf c=5 mg/ml konzentriert, mit Saccharose ad 80 mg/ml aufgestockt und lyophilisiert.

**Beispiel 2**

Herstellung von Polymerteilchen, deren Kern aus einem Streptavidin-Polymerisat und deren Umhüllung aus zwei Schichten Rinderserumalbumin (RSA) besteht.

Wie unter Beispiel 1 beschrieben hergestelltes SA(P)-Lyophilisat wird mit Wasser ad 25 mg/ml gelöst und der pH auf 7,0 eingestellt. 2 ml der erhaltenen SA(P)-Lösung werden mit 2,6 mg MHS (vorgelöst in DMSO) versetzt und unter Rühren 60 Minuten bei 25°C inkubiert. Nach Zugabe von 20 $\mu$l 1 mol/l Lysin wird gegen 10 mmol/l KPP/100 mmol/l NaCl/2 mmol/l EDTA, pH 6,2, dialysiert. Das Dialysat wird mit 5 ml RSA-Lösung (100 mg/ml RSA in 10 mmol/l KPP/100 mmol/l NaCl/2 mmol/l EDTA, pH 6,9; RSA:SA(P)=10:1 (mg/mg)) versetzt, der pH des Reaktionsgemisches auf 6,9 eingestellt und 2 Stunden bei 25°C inkubiert. Nach Zugabe von 44 $\mu$l 0,2 mol/l Cystein-Lösung wird RSA-SA(P) mittels Gelfiltration an Acrylamidagarose von nicht umgesetzten RSA gereinigt. 7,5 mg des auf diese Weise erhaltenen RSA-SA(P) in 1,95 ml 50 mmol/l KPP/100 mmol/l NaCl/2 % Saccharose, pH 7,0, werden mit 770 $\mu$g MHS, vorgelöst in DMSO, versetzt und eine Stunde bei 25°C inkubiert. Danach werden 20 $\mu$l 1 mmol/l Lysin zugegeben und gegen 10 mmol/l KPP/100 mmol/l NaCl/2 mmol/l EDTA, pH 6,2, dialysiert. 1,76 ml des Dialysats (= 5 mg RSA-SA(P), MH-aktiviert) werden mit 1,1 ml der oben beschriebenen RSA-Lösung versetzt, der pH auf 6,9 eingestellt und bei 25°C inkubiert (RSA:RSA-SA(P)= 10:1 mg/mg). Danach werden 29 $\mu$l einer 0,2 mmol/l Cysteinlösung zugegeben und 30 Minuten bei 25°C inkubiert. Das erhaltene RSA-RSA-SA(P) wird mittels Gelfiltration (AcA 22, 50 mmol/l KPP/100 mmol/l NaCl/2 % Saccharose, pH 7,5) von nicht-umgesetzten RSA abgetrennt. Die RSA-RSA-SA(P) enthaltenden Fraktionen werden gesammelt und mittels Ultrafiltration auf eine Konzentration von ca. 1 mg/ml eingeengt.

**Beispiel 3**

Herstellung von Polymerteilchen, deren Kern aus einem Streptavidin-Polymerisat und deren Umhüllung aus zwei sequentiell aufgetragenen Schichten Fab'-Fragment besteht.

Es wird analog zu Beispiel 2 verfahren, wobei anstelle von RSA Fab'-Fragmente eines monoklonalen Anti-TSH-Antikörpers verwendet werden. Fab' und SA(P) bzw. Fab' und Fab'-SA(P) werden dabei im Verhältnis 5:1 (mg/ml) eingesetzt. Ansonsten sind die Reaktionsbedingungen identisch.

Die Hybridoma-Zellinie, die den Anti-TSH-Antikörper produziert, ist bei der European Collection of Animal Cell Cultures, GB unter der Bezeichnung ECACC 87122202 hinterlegt.

**Beispiel 4**

Herstellung von Polymerteilchen, deren Kern aus Streptavidin-Polymerisat und deren Umhüllung aus Aminodextran besteht.

Wie unter Beispiel 2 beschrieben, wird SA(P) mit MHS umgesetzt und im Anschluß gegen 50 mmol/l KPP/100 mmol/l NaCl/2 mmol/l EDTA, pH 6,2 dialysiert.

250 mg Aminodextran (MG 20.000) werden in 10 ml 100 mmol/l KPP, pH 7,3, gelöst. Danach werden 2,9 mg SATA, vorgelöst in DMSO, zugegeben und eine Stunde bei 25°C inkubiert. Nach Zugabe einer 1 mol/l Lysinlösung ad 10 mmol/l wird der pH der Reaktionsmischung auf 6,3 eingestellt und gegen 50 mmol/l KPP/100 mmol/l NaCl/2 mmol/l EDTA, pH 6,2, dialysiert. Dem Dialysat wird 1 mol/l Hydroxylamin, pH 6,2, ad 50 mmol/l zugesetzt und 30 Minuten bei 25°C inkubiert. Danach wird die Reaktionsmischung zweimal eine Stunde gegen je 1 l 50 mmol/l KPP/100 mmol/l NaCl/2 mmol/l EDTA, pH 6,2, dialysiert. Das Dialysat wird mit 10 ml SA(P)-MH-Lösung in Dialysepuffer (c=1 mg/ml) versetzt, der pH sofort auf 6,9 eingestellt und eine Stunde bei 25°C inkubiert. Anschließend wird Cystein ad 1 mmol/l zugegeben und 30 Minuten bei 25°C inkubiert. Danach wird lodacetamid ad 5 mmol/l zugegeben und erneut 30 Minuten bei 25°C inkubiert. Das erhaltene Aminodextran-Streptavidin-Copolymerisat wird eingeengt und mittels Gelfiltration (Acrylamidagarose, 50 mmol/l KPP/100 mmol/l NaCl/2 % Saccharose, pH 7,5) von nicht umgesetztem Aminodextran abgetrennt. Die Aminodextran-Streptavidin-Copolymer-haltigen Fraktionen werden vereinigt und auf eine Konzentration von ca. 1 mg/ml eingeengt.

**Beispiel 5**

Beseitigung der Biotinstörung im CEA-Test (Test auf carcinoembrionales Antigen) durch Fab'-Fab'-SA(P)

Testvorbereitung:

CEA-haltiges Pferdeserum (50 ng CEA/ml) wird mit 50, 100 und 200 ng/ml Biotin aufgestockt. Die Reagenzlösung, enthaltend ein Konjugat aus Fab-Fragmenten von monoklonalen Antikörpern (IgG) gegen CEA mit Peroxidase (POD) sowie biotinylierte monoklonale Antikörper (IgG) gegen CEA in 50 mmol/l 2-(N-Morpholino)-ethansulfonsäurepuffer, pH 6,1, wird mit 5 bzw. 7 µg/ml Fab'-Fab'-SA(P) (hergestellt wie unter Beispiel 3 gezeigt) aufgestockt.

Der Test wurde nach der Vorschrift des im Handel erhältlichen Testkits Enzymun[R] Test CEA (Boehringer Mannheim, Best.Nr. 1132814) durchgeführt.

Testdurchführung:

100 µl CEA-haltiges Pferdeserum werden zusammen mit 1 ml der Reagenzlösung, die Peroxidase-markierte monoklonale Anti-CEA-Antikörper (20 mU POD/ml) und Biotin-markierte monoklonale CEA-Antikörper (1,5 µg/ml) enthält, in ein Streptavidin beschichtetes Luranröhrchen pipettiert und 2 Stunden bei 25°C inkubiert. Dabei bildet sich ein Komplex aus Peroxidase-markiertem CEA-Antikörper, CEA und Biotin-markiertem CEA-Antikörper, der durch Bindung des Biotins an das an der Festphase gebundene Streptavidin immobilisiert wird. Der Röhrcheninhalt wird danach ausgesaugt, dreimal gespült und wiederum sorgfältig ausgesaugt. Danach wird der Anteil an gebundener Peroxidase-Aktivität bestimmt durch Zugabe von 1 ml einer Substratlösung, die 1,9 mmol/l ATBS[R]-Lösung (2,2'-Azino-di-/3-ethylbenzthiazolin-sulfonat(6)/ in 100 mmol/l Phosphat-Citratpuffer/3,2 mmol/l Natriumperborat, pH 4,4, enthält, zugegeben und eine Stunde bei 25°C inkubiert. Es wird dann die Extinktion mit Probe bei 405 nm gegen die Extinktion der Substrat-Chromogen-Lösung als Leerwert gemessen.

Ergebnis:

Wie in Tabelle 1 gezeigt, kann durch Zusatz von Fab'-Fab'-SA(P) die Biotinstörung in diesem Testsystem bis 100 ng/ml Biotin in der Probe quantitativ beseitigt und bei höheren Biotinkonzentrationen deutlich verringert werden.

**Beispiel 6**

Beseitigung der Biotin-Störung in einem Test zum Nachweis von Anti-HBc-Antikörpern (Antikörper gegen Hepatitis B core-Antigen, EP-B-0013828) durch RSA-RSA-SA(P)

Testvorbereitung:

Anti-HBc-Antikörper-freies Serum wird mit 50, 100 und 200 ng/ml Biotin aufgestockt. Eine Lösung, enthaltend ein HBc-Antigen(HBcAg)-Biotin-Konjugat (hergestellt durch Reaktion von HBc Ag mit N-Hydroxysuccinimid-aktiviertem Biotin) in 40 mmol/l Phosphatpuffer, pH 7,4, wird mit RSA-RSA-SA(P), das wie in Beispiel 2 beschrieben, hergestellt wurde, ad 7,5 bis 12,5 µg/ml aufgestockt.

Testdurchführung:

Die Bestimmung wird in einem 2-Schritt-Assay nach dem Kompetitionsprinzip durchgeführt. Als Festphase werden mit Thermo-RSA-Streptavidin (Herstellung nach EP-A 0269092) beschichtete Kunststoffröhrchen verwendet. Als markierte Rezeptoren werden Anti-HBc-Antikörper-Peroxidase-Konjugate eingesetzt.

200 µl eines von anti-HBc-Antikörper-freiem Serum werden zusammen mit 1 ml einer Lösung, die Biotin-HBcAg-Konjugat (10 ng/ml) enthält, in ein mit Thermo-RSA-Streptavidin beschichtetes Luranröhrchen pipettiert und eine Stunde bei 25°C inkubiert. Dabei reagieren die anti-HBc-Antikörper aus der Probe mit dem Biotin-HBcAg-Konjugat und gebildete Komplexe werden durch Bindung des Biotins aus dem Konjugat an das festphasengebundene Streptavidin immobilisiert. Danach wird der Röhrcheninhalt ausgesaugt und das Röhrchen dreimal mit Wasser gewaschen. Dabei werden nicht gebundenes biotinyliertes HBcAg und sämtliche Serumbestandteile entfernt. Anschließend wird 1 ml einer Lösung, die Peroxidase markierte anti-HBcAg-Antikörper enthält (200 mU Peroxidase/ml), zugegeben und eine Stunde bei 25°C inkubiert. Dabei binden Peroxidase-markierte anti-HBc-Antikörper mit durch von der Probe nicht besetzten Antigen-Bindungsstellen. Anschließend wird der Röhrcheninhalt ausgesaugt und erneut dreimal gewaschen, wobei nicht gebundenes Peroxidase-Konjugat entfernt wird. Danach werden 1 ml 1,9 mmol/l ABTS-Lösung in 100 mmol/l Phosphat-Citratpuffer/3,2 mmol/l Natriumperborat, pH 4,4, zugegeben und eine Stunde bei 25°C inkubiert. Anschließend wird die Extinktion mit Probe bei 405 nm gegen die Extinktion der Substrat-Chromogen-Lösung (Leerwert) bestimmt).

Wie in Tabelle 2 gezeigt ist, läßt sich durch Zugabe von RSA-RSA-SA(P) zu diesem Testsystem die Biotinstörung bis 200 ng/ml nahezu vollständig beseitigen.

**Beispiel 7**

Herstellung von Polymerteilchen, deren Kern aus Rinderserumalbumin-Streptavidin-Copolymerisat und deren Umhüllung aus Fab'-Fragmenten besteht.

Analog zu Beispiel 2 wird zunächst RSA-SA(P) hergestellt. Dieses Copolymerisat wird, wie im Beispiel 2 beschrieben, mit MHS aktiviert. Das RSA-SA(P)-MH wird mit Fab-Fragmenten von Anti-TSH-Antikörper (vgl. Beispiel 3) im Verhältnis 5:1 (mg/mg) umgesetzt unter den im Beispiel 2 beschriebenen Reaktionsbedingungen.

**Beispiel 8**

Beseitigung der Biotinstörung in einem Test zum Nachweis von Anti-HBc-Antikörpern durch Fab'-RSA-SA(P).

Der Test wurde wie im Beispiel 6 beschrieben durchgeführt, wobei jedoch die HBcAg-Biotin-Lösung mit 10 und 15 µg/ml Fab'-RSA-SA(P) aufgestockt wurde. Serum und HBcAg-Biotin-Lösung werden nicht zusammen in das Streptavidin-beschichtete Luranröhrchen pipettiert, sondern es wird zunächst 200 µl Serum in das Röhrchen gegeben und erst nach einem Zeitintervall von 2 bis 5 Minuten die HBcAg-Biotin-Konjugat-Lösung hinzugefügt.

Tabelle 3 zeigt, daß bei dieser geänderten Testführung ebenfalls eine deutliche Entstörung zu beobachten ist.

Tabelle 1

| Biotin-zusatz ng/ml | % des Ausgangswerts[1] | | |
|---|---|---|---|
| | ohne Polymer | 5 $\mu$g/ml Polymer | 7 $\mu$g/ml Polymer |
| 0 | 100 | 92 | 85 |
| 50 | 78 | 92 | 84 |
| 100 | 46 | 85 | 82 |
| 200 | 20 | 43 | 60 |

[1] Ausgangswert: Extinktion (abzüglich Leerwert) mit Probe ohne Biotin und ohne Zusatz von Polymerteilchen

Tabelle 2

| Biotin-zusatz ng/ml | % des Ausgangswerts[1] | | | |
|---|---|---|---|---|
| | ohne Polymer | 7,5 $\mu$g/ml Polymer | 10 $\mu$g/ml Polymer | 12,5 $\mu$g/ml Polymer |
| 0 | 100 | 93 | 90 | 89 |
| 50 | 74 | 93 | 90 | 87 |
| 100 | 50 | 91 | 86 | 85 |
| 200 | 24 | 55 | 70 | 78 |

[1] Ausgangswert: Extinktion (abzüglich Leerwert) mit Probe ohne Biotin und ohne Zusatz von Polymerteilchen

Tabelle 3

| Biotin-zusatz ng/ml | % des Ausgangswerts[1] | | |
|---|---|---|---|
| | ohne Polymer | 10 $\mu$g/ml Polymer | 15 $\mu$g/ml Polymer |
| 0 | 100 | 85 | 80 |
| 100 | 52 | 74 | 69 |
| 200 | 28 | 50 | 67 |

[1] Ausgangswert: Extinktion (abzüglich Leerwert) mit Probe ohne Biotin und ohne Zusatz von Polymerteilchen

## Patentansprüche

1. Verfahren zum Nachweis spezifisch bindefähiger Substanzen in Körperflüssigkeiten nach dem Prinzip des Immunoassay unter Verwendung von Ligandenkonjugaten zur Markierung, Immobilisierung oder Komplexierung, die die für die Ligandenkonjugate verwendeten Liganden in freier Form enthalten, **dadurch gekennzeichnet,** daß man die in freier Form vorliegenden Liganden der immunologischen Reaktion entzieht, indem man die Probelösung mit Polymerteilchen, bestehend aus Kern und Umhüllung, inkubiert, die als Kern ein Polymer, das eine Vielzahl von Bindungsstellen für den Liganden aufweist und als Umhüllung mindestens eine Schicht aus Protein, Peptid, Nukleinsäurepolymer, Kohlenhydrat und/oder einem Copolymerisat aus Kohlenhydrat und Aminosäuren haben, wobei die Schichtdicke der Umhüllung so eingestellt wird, daß die Beschichtung durchlässig für den freien Liganden, nicht jedoch für das Ligandenkonjugat ist.

2. Verfahren nach Anspruch 1, **dadurch gekennzeichnet,** daß man das Kernpolymer mit zwei oder mehr Schichten umhüllt.

3. Verfahren nach Anspruch 2, **dadurch gekennzeichnet,** daß man für die Umhüllung mindestens zwei voneinander verschiedene Schichten verwendet.

4. Verfahren nach einem der vorhergehenden Ansprüche, **dadurch gekennzeichnet,** daß man für die Umhüllung Rinderserumalbumin, Antikörper oder deren Fragmente und/oder Kohlenhydrat verwendet.

5. Verfahren nach einem der vorhergehenden Ansprüche,

**dadurch gekennzeichnet,**
daß man als Kern ein Polymerisat aus einer mit dem Liganden spezifisch bindefähigen Substanz und einer, bezüglich der immunologischen Bindung inerten Substanz verwendet.

6.  Verfahren nach einem der vorhergehenden Ansprüche,
    **dadurch gekennzeichnet,**
    daß man als Kern homogen oder heterogen vernetztes polymeres Streptavidin oder Avidin verwendet.

7.  Verfahren nach einem der vorhergehenden Ansprüche,
    **dadurch gekennzeichnet,**
    daß Biotin der Ligand ist und der Kern ein Polymerisat aus Streptavidin, Avidin oder Antibiotin-Antikörpern enthält oder daraus besteht.

8.  Ligandenfänger,
    **dadurch gekennzeichnet,**
    daß er aus einem wasserlöslichen polymeren Rezeptor-Molekül, das Kern und Umhüllung umfaßt, besteht, wobei der Kern ein Polymer ist, das eine Vielzahl von Bindungsstellen für den Liganden aufweist und die Umhüllung aus mindestens einer Schicht aus Protein, Peptid, Nukleinsäurepolymer, Kohlenhydrat und/oder einem Copolymerisat aus Kohlenhydrat und Aminosäure besteht, wobei die Schichtdicke der Umhüllung so ist, daß die Beschichtung durchlässig für den freien Liganden, nicht jedoch für ein Ligandenkonjugat ist.

9.  Ligandenfänger nach Anspruch 8,
    **dadurch gekennzeichnet,**
    daß der Kern aus einem homogenen oder heterogenen Avidin- oder Streptavidin-Polymerisat besteht und die Umhüllung aus mindestens einer Schicht Rinderserumalbumin, Antikörper und/oder Kohlenhydrat-Polymer besteht.

## Claims

1.  Process for the detection of specifically- bindable substances in body fluids according to the immunoassay principle with use of ligand conjugates for the labelling, immobilisation or complexing which contain in free form the ligands used for the ligand conjugates, characterised in that one removes the ligands present in free form from the immunological reaction in that one incubates the sample solution with polymer particles consisting of core and covering which, as core, have a polymer which has a plurality of binding positions for the ligands and, as covering, at least one layer of protein, peptide, nucleic acid polymer, carbohydrate and/or a co-polymer of carbohydrate and amino acids, whereby the layer thickness of the covering is so adjusted that the coating is permeable for the free ligands but not for the ligand conjugate.

2.  Process according to claim 1, characterised in that one coats the core polymer with two or more layers.

3.  Process according to claim 2, characterised in that, for the covering, one uses at least two layers different from one another.

4.  Process according to one of the preceding claims, characterised in that one uses bovine serum albumin, antibodies or their fragments and/or carbohydrate for the covering.

5.  Process according to one of the preceding claims, characterised in that, as core, one uses a polymer of a substance specifically bindable with the ligands and a substance inert with regard to the immunological binding.

6.  Process according to one of the preceding claims, characterised in that, as core, one uses homogeneously or heterogeneously cross-linked polymeric streptavidin or avidin.

7.  Process according to one of the preceding claims, characterised in that biotin is the ligand and the core contains or consists of a polymer of streptavidin, avidin or anti-biotin antibodies.

8.  Ligand trap, characterised in that it consists of a water-soluble polymeric receptor molecule which includes core and covering, whereby the core is a polymer which has a plurality of binding positions for the ligands and the covering consists of at least one layer of protein, peptide, nucleic acid polymer, carbohydrate and/or a co-polymer of carbohydrate and amino acid, whereby the layer thickness of the covering is such that the covering is permeable for the free ligands but not for a ligand conjugate.

9. Ligand trap according to claim 8, characterised in that the core consists of a homogeneous or heterogeneous avidin or streptavidin polymer and the covering consists of at least one layer of bovine serum albumin, antibody and/or carbohydrate polymer.

## Revendications

1. Procédé de mise en évidence de substances capables de se lier spécifiquement dans les fluides corporels selon le principe des immuno-analyses à l'aide de conjugués de ligands pour le marquage, l'immobilisation ou la complexation, qui contiennent les ligands utilisés pour les conjugués de ligands sous forme libre, caractérisé en ce que l'on soustrait de la réaction immunologique les ligands qui sont sous forme libre en incubant la solution échantillon avec des particules de polymère, consistant en un noyau et en une enveloppe, qui comportent comme noyau un polymère qui présente une multiplicité de sites de liaison pour le ligand et comme enveloppe au moins une couche de protéine, de peptide, de polymère d'acide nucléique, de glucide et/ou d'un copolymère de glucide et d'acides aminés, l'épaisseur de couche de l'enveloppe étant choisie de manière que le revêtement soit perméable au ligand libre mais pas au conjugué de ligand.

2. Procédé selon la revendication 1, caractérisé en ce que l'on recouvre le polymère du noyau de deux couches ou plus.

3. Procédé selon la revendication 2, caractérisé en ce que l'on utilise pour l'enveloppe au moins deux couches différentes l'une de l'autre.

4. Procédé selon l'une des revendications précédentes, caractérisé en ce que l'on utilise pour l'enveloppe la sérumalbumine humaine, des anticorps ou des fragments d'anticorps et/ou un glucide.

5. Procédé selon l'une des revendications précédentes, caractérisé en ce que l'on utilise comme noyau un polymère d'une substance capable de se lier spécifiquement au ligand et d'une substance inerte vis à vis de la liaison immunologique.

6. Procédé selon l'une des revendications précédentes, caractérisé en ce que l'on utilise comme noyau la streptavidine ou l'avidine polymérique réticulée de manière homogène ou hétérogène.

7. Procédé selon l'une des revendications précédentes, caractérisé en ce que la biotine est le ligand et le noyau contient un polymère de streptavidine, d'avidine ou d'anticorps antibiotine ou est constitué par un tel polymère.

8. Capteur de ligand, caractérisé en ce qu'il consiste en une molécule de récepteur polymérique hydrosoluble comportant un noyau et une enveloppe, le noyau étant un polymère qui présente une multiplicité de sites de liaison pour le ligand et l'enveloppe consistant en au moins une couche de protéine, de peptide, de polymère d'acide nucléique, de glucide et/ou d'un copolymère de glucide et d'acide aminé, l'épaisseur de couche de l'enveloppe étant telle que le revêtement est perméable au ligand libre mais pas à un conjugué de ligand.

9. Capteur de ligand selon la revendication 8, caractérisé en ce que le noyau consiste en un polymère homogène ou hétérogène d'avidine ou de streptavidine et l'enveloppe consiste en au moins une couche de sérumalbumine bovine, d'anticorps et/ou de polymère de glucide.

# Fig.1a

# Fig.1b

# Fig.2a

# Fig.2b